# EUROPEAN PATENT APPLICATION

(11) **EP 0 619 135 A1**
(43) Date of publication of application: **12.10.1994**
(21) Application number: 94830162.7
(22) Date of filing: 05.04.1994
(51) Int. Cl.: B01F 5/10, A61M 1/16

(54) **System for preparing fluid for medical treatment**

(30) Priority: 05.04.1993 IT TO930229
(71) Applicant: S.I.F.RA. SOCIETA ITALIANA FARMACEUTICI RAVIZZA S.p.A., I-37063 Isola Della Scala (IT)
(72) Inventor: Fecondini, Luciano, I-40100 Bologna (IT)
(74) Representative: Boggio, Luigi

(57) **Abstract**

A system for preparing fluid for medical treatment by dissolving a first compound in a second compound; the system including a disposable hydraulic circuit (4) presenting a container (8) containing the first compound, a first tube (18) for feeding the second compound to the container (8), and a tube (22) external to the container (8) and presenting two end portions (22b, 22c) communicating with the container (8); the system also including an ultrasonic generator (6') external to the hydraulic circuit (4), for assisting dissolution of the first compound in the second compound; and pumping means (34) fitted to the external tube (22), for recirculating the fluid to and from the container (8).

## Description

The present invention relates to a system for preparing fluid for medical treatment.

Various systems are known for preparing fluid for medical treatment, each of which normally presents a hydraulic circuit, sensor means, and an electronic apparatus to which the sensor means are connected and which provides for controlling the hydraulic circuit. Such systems operate differently and present specific component configurations according to the type of fluid to be prepared and the treatment involved, which basically means a system suitable for one fluid is unsuitable for another.

Moreover, practically all the components of known systems are fixed, i.e. used repeatedly for more than one preparation cycle, so that they must be sterilized after each cycle with all the disadvantages this entails, such as the high cost of the sterilizing equipment and the downtime involved for the actual sterilizing operation.

Publication DE-A-491 981 relates to a fixed mixer to which the two compounds of a dialysis fluid are supplied, and featuring a circuit for recirculating the fluid, the preparation process being controlled by a processing unit on the basis of sensor-detected parameters. A major drawback of this system is that it fails to provide for rapidly dissolving the two compounds.

It is an object of the present invention to provide a system and process for preparing fluid for medical treatment, designed to overcome the aforementioned drawbacks, i.e. which provides for rapidly dissolving the compounds, preparing different fluids for different medical treatments, and eliminating sterilization of at least some of the components.

According to the present invention, there is provided a system for preparing fluid for medical treatment by dissolving a first chemical compound in a second chemical compound; the system comprising a hydraulic circuit with a container to which are connected a first tube by which a predetermined amount of said second compound is channelled to the container, a second tube by which, at the end of the preparation cycle, the prepared fluid is channelled from the container to a user device, and a third tube presenting a portion external to the container and two end portions communicating with the container, and comprising pumping means fitted to said external portion, for recirculating the fluid being prepared to and from the container; characterized in that a predetermined amount of said first compound is contained inside the container; and that a generator for generating vibrations of over 10 kHz frequency is fitted outside a given portion of said circuit, for assisting dissolution of said first compound in said second compound.

According to the present invention, there is also provided a process for preparing fluid for medical treatment by dissolving a first compound in a second compound; said first compound being contained inside a container; characterized in that it comprises, in succession, at least the following stages:
a stage wherein a predetermined amount of said second compound is fed into the container;
a stage wherein the fluid is vibrated continually and recirculated into the container along a tube external to the container; and
a stage for checking attainment of the required characteristics of said fluid.

A number of preferred, non-limiting embodiments of the present invention will be described by way of example with reference to the accompanying drawings, in which:
Figure 1 shows a first embodiment of a system for preparing fluid for medical treatment in accordance with the present invention;
Figure 2 shows a block diagram of an electronic apparatus for controlling the Figure 1 system;
Figure 3 shows an operational flow chart of the Figure 1 system;
Figures 4 and 5 show a side view and section respectively of a component of the Figure 1 system in the cycle-start configuration;
Figure 6 shows a partial view of a variation of the Figure 1 system;
Figure 7 shows a second embodiment of the system according to the present invention;
Figure 8 shows a vertical section of a detail of the Figure 7 system;
Figure 9 shows a section along line IX-IX in Figure 8;
Figure 10 shows a section along line X-X in Figure 8 in a different operating position;
Figure 11 shows a larger-scale partial section of a variation of the Figure 8 detail;
Figure 12 shows a partial view of a variation of the Figure 7 system.

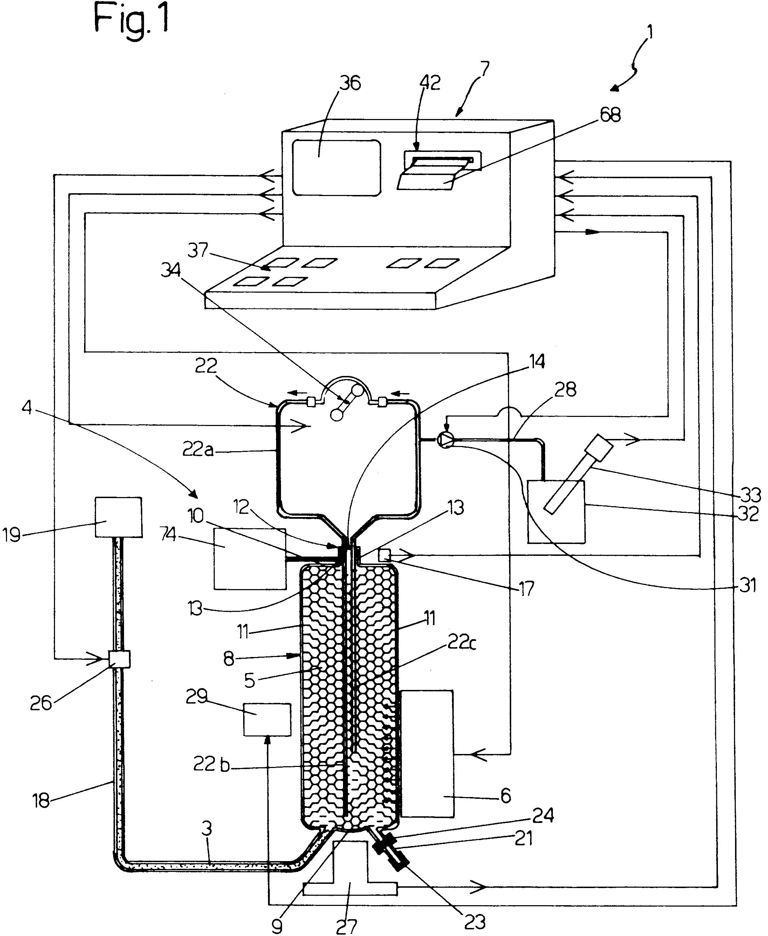

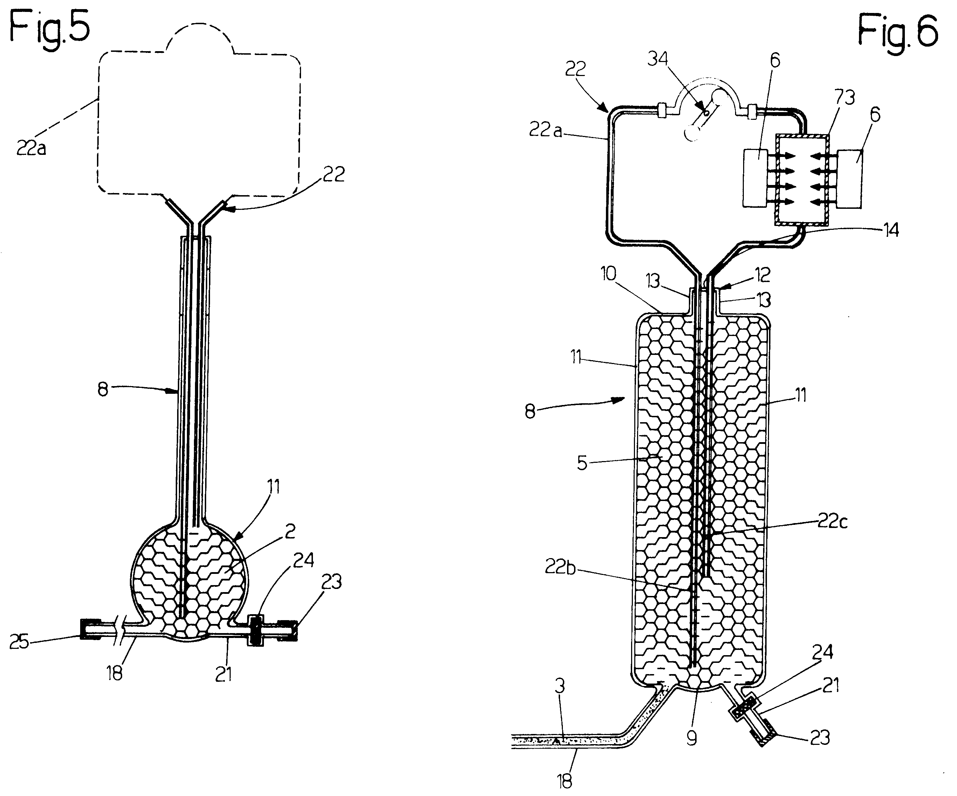

Number 1 in Figure 1 indicates a system for preparing fluid for medical treatment by dissolving a first chemical compound 2 (shown in Figure 5 and preferably soluble) in a second chemical compound 3, usually distilled, demineralized water. System 1 is particularly suitable (though this in no way limits the scope of the present invention) for preparing haemodialysis, haemodiafiltration, haemofiltration, peritoneal dialysis fluids and infusion solutions.

System 1 comprises a hydraulic circuit 4; a number of sensors, described further on, for detecting parameters and quantities in circuit 4 relative to fluid 5 being prepared; a vibration generator 6 for generating vibrations of over 10 kHz frequency, for assisting dissolution of first compound 2 in second compound 3, and which is advantageously selected to generate vibrations of 10 to 40 kHz frequency; and an electronic apparatus 7 to which said sensors and a number of actuators are connected, and which provides for controlling operation of generator 6, supply of second compound 3, and recirculation of fluid 5 being prepared.

Hydraulic circuit 4 may preferably be what is referred to by technicians as "disposable", i.e. used for only one preparation cycle, and comprises an expansible container 8 made of plastic material, and which, in its initial configuration (Figures 4 and 5), presents a lower portion 11 containing first compound 2 in the form of dry or wet powder, granules or liquid.

To reduce its initial size, a vacuum is preferably formed inside container 8 in its initial configuration. During preparation of the fluid, container 8 in the fully expanded configuration shown in Figure 1 is box-shaped, and presents a bottom wall 9, a top wall 10, and four side walls 11, the opposite pairs of which are parallel.

In top wall 10 of container 8, there is formed an upward extension 12 defined by two small side walls 13 and a small top wall 14. As shown in Figure 4, two labels 15 and 16 are applied to the outer surface of one of side walls 13 of extension 12, label 15 bearing writing legible by the operator and indicating the type of compounds 2 and 3, and the type of fluid 5 to be prepared, and label 16 indicating the same information as label 15, but in the form of a code legible by a reading unit 17 connected to apparatus 7.

Hydraulic circuit 4 also comprises a first tube 18 extending from a source 19 of second compound 3 and connected to bottom wall 9 of container 8; a second tube 21 extending from bottom wall 9 of container 8, for channelling fluid 5 prepared by system 1 to a user device indicated schematically by 74; and a third tube 22 presenting a central portion 22a external to container 8, and two end portions 22b, 22c connected to container 8.

More specifically, in the Figure 1 embodiment and the variation shown in Figure 6, the two end portions 22b, 22c are inserted downwards through wall 14 of extension 12 into container 8; end portion 22b, which acts as the inlet of container 8, is longer than end portion 22c which acts as the outlet; and end portion 22b terminates close to bottom wall 9 of container 8, while end portion 22c terminates in a central portion of container 8.

When preparing fluid 5, tube 21 is closed by a leaktight plug 23, and fitted with a filtering member 24; and in actual use, before fitting circuit 4 to system 1, i.e. before connecting tube 18 to source 19, tube 18 (Figure 5) is also closed by a leaktight plug 25.

With reference to Figure 1, system 1 also comprises:
a solenoid valve 26 fitted along tube 18, and controlled by apparatus 7 for disabling or enabling flow of compound 3 along tube 18;
means 27 for weighing container 8 and transmitting said weight to apparatus 7;
a branch tube 28 extending from portion 22a of tube 22;
a pump 31 fitted to tube 28 and controlled by apparatus 7;
a burette 32 in which the free end of tube 28 terminates;
a sensor 33 inside burette 32, for detecting and transmitting to apparatus 7 the parameter values of fluid 5 being prepared;
a peristaltic pump 34 fitted to a portion of portion 22a of tube 22 and controlled by apparatus 7; and
a heating device 29 adjacent to container 8, for heating fluid 5, and controlled by apparatus 7.

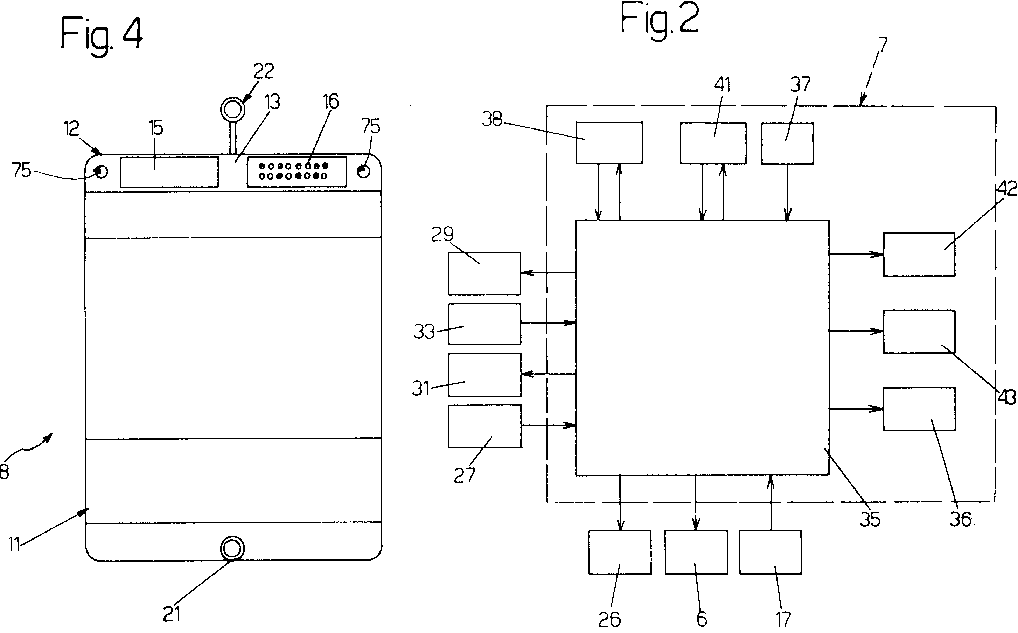

With reference to Figures 1 and 2, apparatus 7 comprises a central data processing unit 35 to which are connected a digital display 36; a data entry and control keyboard 37; a memory unit 38 in which the data and control program for preparing each type of fluid are stored; a timer unit 41 for setting a minimum operating time for generator 6 and pump 34; a label printer 42; and an acoustic signal 43.

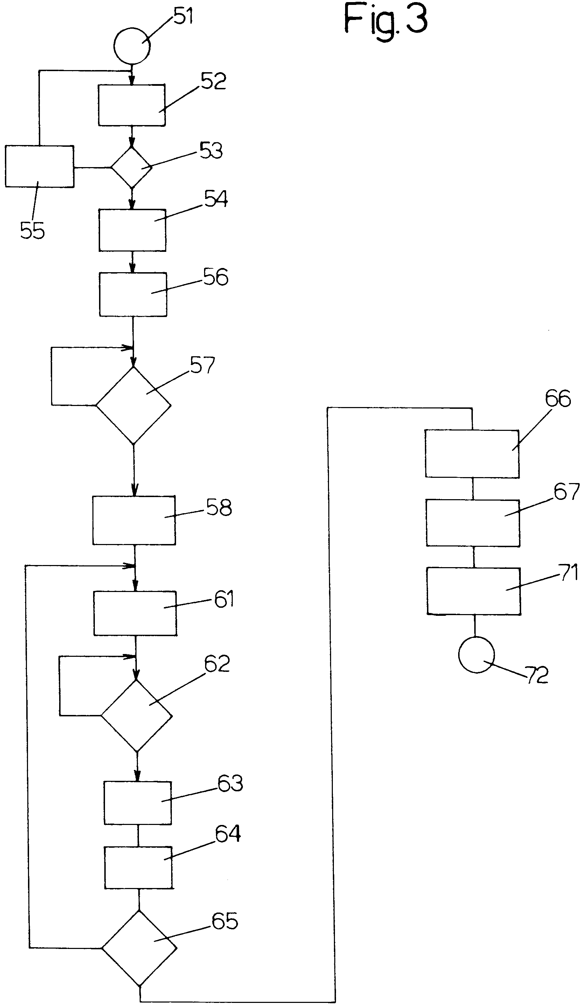

Operation of system 1 in Figures 1 to 5 will now be described with reference to the flow chart in Figure 3 wherein a start block 51 goes on to a block 52 in which apparatus 7 reads the code on label 16 by means of unit 17, provides for displaying the reading on display 36, and requests the operator to confirm the display reading and continuation of the preparation process.

The display indicates the type of treatment in which fluid 5 is to be used, the type of fluid 5 to be prepared, the type of compound 2 and 3, and the end weight (and hence quantity) of fluid 5 to be prepared. As a predetermined time is of course allotted for preparing each fluid, this is also shown on the display.

Block 52 then goes on to block 53 in which the operator confirms the display reading and continuation of the preparation process by pressing a first key on keyboard 37, in which case block 53 goes on to block 54; or, conversely, presses a second key on keyboard 37, in which case block 53 goes on to block 55 and from there to block 52. In block 55, the original data shown on display 36 may be altered using further keys on keyboard 37; and, in block 54, unit 35, on the basis of the data entered, identifies the fluid to be prepared, retrieves from memory unit 38 the control data for preparing the fluid, and enables weighing means 27 to determine the tare of container 8.

The functions performed by apparatus 7 may be implemented by means of a special program or prewired circuits, and all the data relative to preparation of the fluid for a given patient, i.e. the stoichiometric strength of the fluid best suited to the patient, may be stored in memory unit 38 and retrieved by block 54 of unit 35 by entering the identification data of the patient on apparatus 7.

Fluid 5, or rather the manner in which the fluid is prepared, may thus be adapted to take into account specific requirements of the patient. For example, in the case of a haemodialysis fluid in which compound 2 is defined by a saline concentrate and compound 3 by water, it is possible to vary the salt concentration in relation to the water.

Block 54 then goes on to block 56 in which unit 35 enables solenoid valve 26 to supply compound 3 from source 19 to container 8; activates pump 34 to recirculate fluid 5; activates device 29; and enables operation of vibration generator 6. More specifically, fluid 5 flows along portion 22a in the direction of the arrows in Figure 1; and pump 34 rotates anticlockwise (in Figure 1) to withdraw fluid 5 along portion 22c of tube 22 and feed it into container 8 along portion 22b.

Generator 6 in turn generates vibrations for dispersing or breaking up the chemical compounds into elementary particles. Dispersion is assisted by device 29 raising the temperature inside container 8; and breaking the compounds up into elementary particles provides for increasing the contact surface of compounds 2 and 3, and so greatly accelerating dissolution of compound 2 in compound 3, and preventing parts of compound 2 from remaining suspended in fluid 5.

Block 56 then goes on to block 57 which, via weighing means 27, determines whether the predetermined weight of container 8 has been reached. If it has, block 57 goes on to block 58; if it has not, block 57 goes back to its own input. Block 58 provides for disabling solenoid valve 26 to cut off supply of compound 3 along tube 18, and then goes on to block 61 in which unit 35 enables timer unit 41.

Block 61 goes on to block 62 which determines whether a predetermined time stored in memory unit 38, and which is typical of fluid 5 and retrieved from unit 38 together with other data in block 54, has been reached. If it has, block 62 goes on to block 63; if it has not, block 62 goes back to its own input. In block 63, unit 35 activates pump 31 to withdraw a predetermined quantity of fluid from container 8 and feed it into burette 32.

Block 63 then goes on to block 64 which enables sensor 33 to determine characteristic fluid parameter values and transmit them to unit 35. For example, in the case of a haemodialysis fluid, sensor 33 may consist of a sensor for determining the conductivity of fluid 5, which, as is known, provides for determining the chemical composition of the fluid. In the case of fluids for other types of treatment, sensor 33 may provide for determining the pH of the fluid.

Block 64 then goes on to block 65 which compares the parameter values detected by sensor 33 with those of the same parameters adopted previously and either retrieved from memory unit 38 or entered by the operator. In the event the values detected by sensor 33 match the previously adopted values, block 65 goes on to block 66 in which unit 35 disables pump 34, device 29 and generator 6. Conversely, block 65 goes back to block 61.

Block 66 then goes on to block 67 in which unit 35 enables printer 42 to print the characteristic data of the prepared fluid, such as the date of preparation, fluid strength, number of preparation cycles, etc., on a label 68 (Figure 1) which is then applied to the outer surface of one of side walls 11 of container 8. Block 67 goes on to block 71, in which unit 35 activates an end-of-preparation acoustic signal 43, and which then goes on to end-of-cycle block 72.

In the Figure 6 variation, hydraulic circuit 4 differs from that of Figure 1 by presenting, along a portion of external portion 22a of tube 22, a box-shaped body 73 through which the recirculating fluid 5 flows; and, adjacent to two side walls of body 73, there are provided respective generators 6 for generating vibrations of 10 to 40 kHz frequency, and which replace the Figure 1 generator 6 adjacent to container 8.

Whichever the case, system 1 comprises a frame 74 - shown schematically in Figure 1 - for supporting circuit 4 and, in particular, container 8 which, on extension 12 (Figure 4), presents two holes 75 engaged by pins extending from frame 74.

To prepare a single dose of haemodialysis fluid consisting of a solution of sodium bicarbonate and water, 400 to 900 grams (preferably 600 grams) of sodium bicarbonate powder is contained in container 8, and 5 to 11.25 liters (preferably 7.5 liters) of water is fed from source 19. Container 8 must therefore be so selected as to accommodate the above quantities when fully expanded.

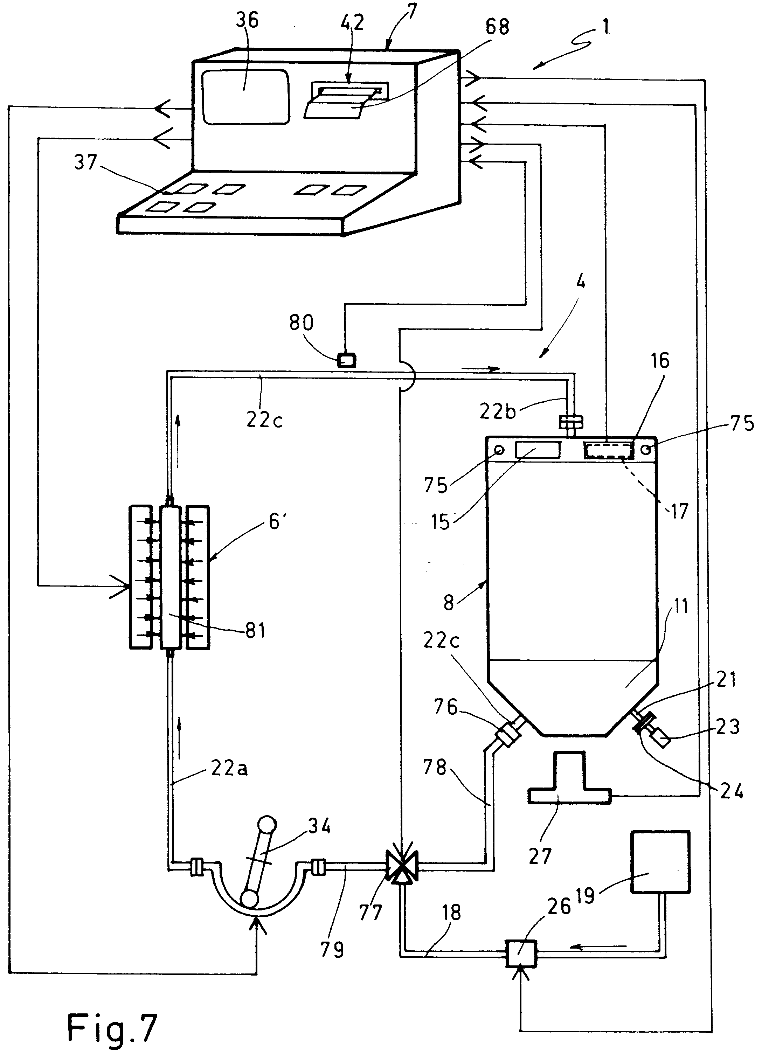

In the Figure 7-12 embodiment - in which the component parts similar to those of Figure 1 are indicated using the same numbering system with no further description - container 8 containing compound 2 is again possibly expansible, and comprises an extension 12 with two labels 15 and 16. In the Figure 7 embodiment, however, only inlet end portion 22b of tube 22 is inserted inside extension 12, the outlet end portion 22c being inserted inside a fitting 76 at the bottom.

Hydraulic circuit 4 comprises a three-way solenoid valve 77 controlled by apparatus 7 and located between two portions 78, 79 of external portion 22a of tube 22; solenoid valve 77 is also connected to tube 18 connecting source 19 of second compound 3; peristaltic pump 34 is rotated clockwise (in Figure 7); and a further portion of tube 22 presents a sensor 80 controlled by apparatus 7, for detecting various characteristics of fluid 5.

More specifically, sensor 80 is located outside portion 22a of tube 22, and provides for optically determining characteristic parameter values of fluid 5, such as colour, opacity, etc., for transmission to apparatus 7. As such, system 1 in Figure 7 may dispense with branch tube 28, pump 31 and burette 32 in the Figure 1 system.

Hydraulic circuit 4 in Figure 7 presents an expansible bag 81 between two portions of external portion 22a of tube 22; and an ultrasonic generator 6' is provided adjacent to bag 81, for generating ultrasonic vibrations of over 100 kHz frequency, and which is preferably selected to generate ultrasonic vibrations of 400 to 1000 kHz frequency.

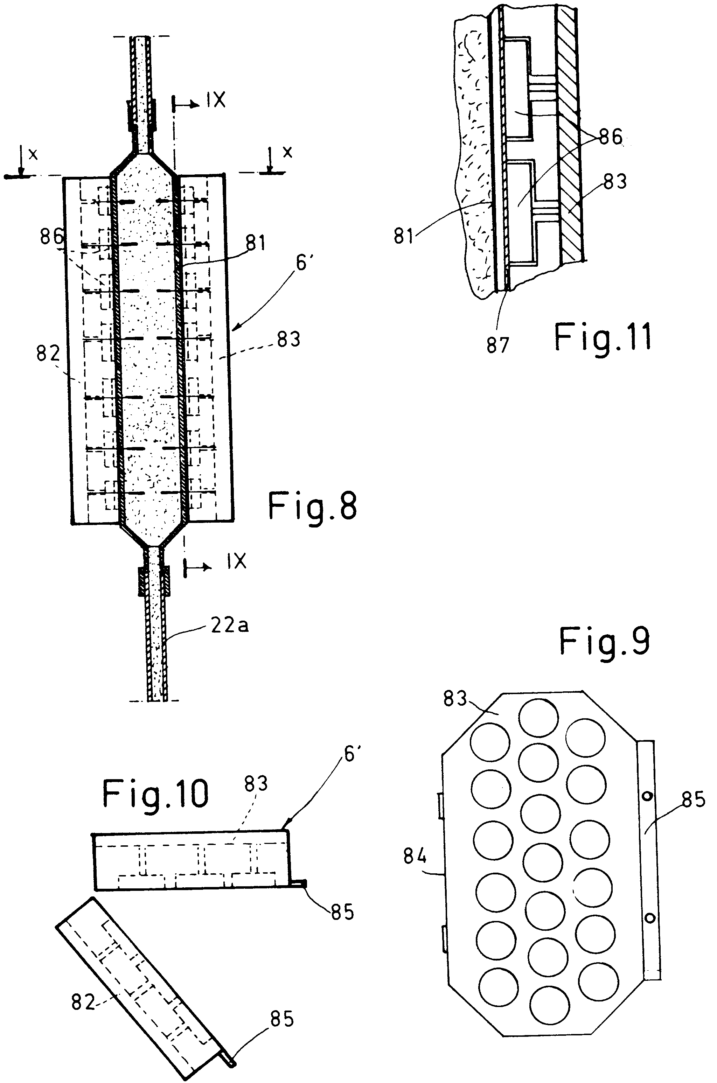

Generator 6' comprises two symmetrical plates 82 and 83 (Figures 8 to 10) hinged along a vertical edge 84 and releasably connected, e.g. by means of screws, along the opposite edge 85 to enable insertion of bag 81 between the two plates. Each plate 82, 83 presents a number of, say, 50 mm diameter piezoelectric ceramic disks 86 which may be arranged in various ways on plate 82, 83, e.g. in rows and columns, and which are preferably arranged in offset rows as shown in Figure 10.

According to the Figure 8-10 variation, disks 86 are arranged facing the walls of bag 81, through which the ultrasonic vibrations are transmitted directly to the fluid. As the fluid circulates inside tube 22, bag 81 expands to contact disks 86. Alternatively, as shown in the Figure 11 variation, each number of disks 86 contacts a metal plate 87 which is contacted by the wall of bag 81 as this expands. In both cases, each disk 86 is driven by a respective 30-40 w self-oscillator (not shown) excited by an appropriate power supply. Excellent test results have been obtained using self-oscillators generating a frequency of 800 kHz.

Apparatus 7 is the same as in Figure 1, except for the substitution of sensor 80 for sensor 33, and the addition of solenoid valve 77; and the preparation cycle is performed in the same way and therefore need not be described in detail. In the Figure 7 embodiment of system 1, solenoid valve 77 is first activated to cut off communication between portions 78 and 79 of external tube portion 22a; and fluid 3 flows from source 19 into portion 79, and is pumped by peristaltic pump 34 into bag 81 which expands to contact disks 86 (Figure 8) or plates 87 (Figure 11).

On achieving the required fluid weight inside container 8, solenoid valve 77 is activated to connect portions 78 and 79 (Figure 7); pump 34 circulates fluid 5 along tube 22 between bag 81 and container 8; and generator 6' is energized to vibrate disks 86 at ultrasonic frequency and convert the electric energy into mechanical energy which is transmitted through the molecules of fluid 5 to the salt crystals to rapidly dissolve the crystals. Tests have shown ultrasonic vibration to be so effective that most salts require no heating of the fluid.

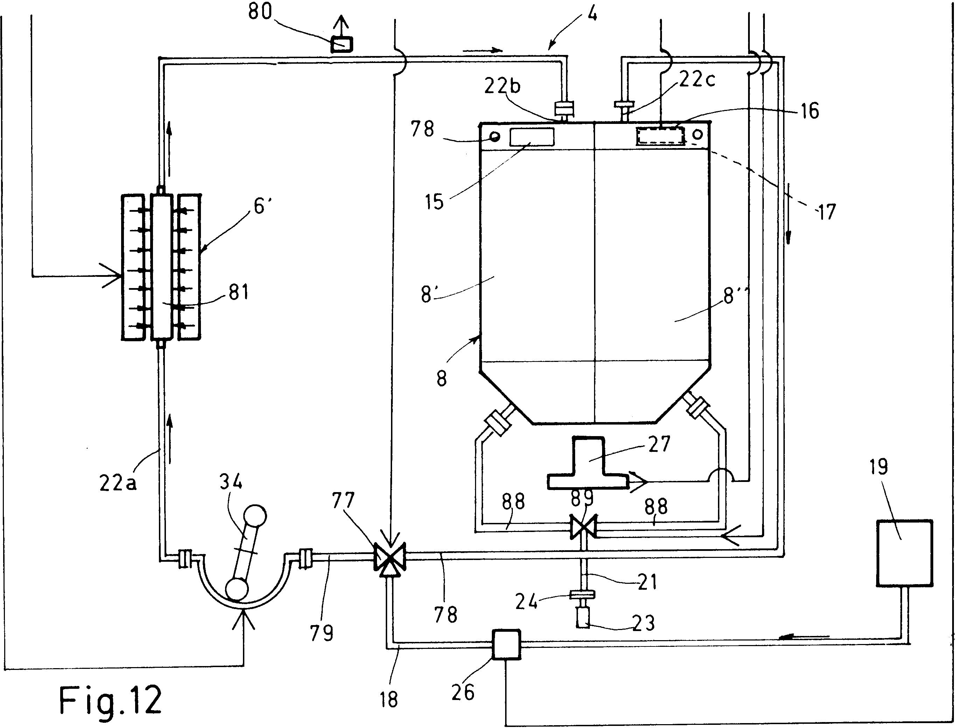

According to the Figure 12 variation, container 8 presents two chambers 8' and 8'' separated by a membrane or by heat sealing the walls of container 8, and containing two different compounds with which to prepare fluid 5 and which interact when brought into contact with each other. In this case, inlet end portion 22b of tube 22 is inserted inside the top wall of chamber 8'; and outlet end portion 22c is inserted inside the top wall of chamber 8'' and connected by portion 78 to three-way solenoid valve 77. Chambers 8' and 8'' are connected to each other by a further tube 88 fitted with a further three-way valve 89 which may be controlled automatically by apparatus 7.

When preparing fluid 5, valve 89 is first set so as to connect the two portions of tube 88 and hence chambers 8' and 8''; and pump 34 and ultrasonic generator 6' are activated to feed compound 3 along tube 22a and through bag 81 into chamber 8' and from there into chamber 8''. On achieving the required weight of fluid 5, valve 77 is activated to recycle fluid 5 for a predetermined time under the control of timer 41 (Figure 2) and sensor 80; after which, valve 89 is activated to feed fluid 5 to the user device.

The advantages of the present invention are as follows. In particular, vibration generator 6, and especially ultrasonic generator 6', provides for greatly improving dissolution of compound 2, and far more rapidly as compared with known techniques.

Medical centers may thus be equipped with apparatus 7, frame 74, and all the components of system 1 connected to apparatus 7, and may purchase sealed disposable hydraulic circuits 4 for fitment to system 1 whenever fluid 5 is required. As such, purchasing and storage is limited to compact circuits 4 and containers 8, unlike the current practice of purchasing and storing large-sized cans of ready-made fluid.

As canned fluid, when stored for prolonged periods of time, is known to be perishable and potentially toxic, storing containers 8 containing only concentrated chemical compound, which is far less subject to deterioration, is undoubtedly an advantage both economically and in terms of safety. As such, system 1 is particularly suitable for preparing unstable fluids subject to rapid deterioration and which must therefore be used immediately.

Moreover system 1 provides for preparing fluids of different strength for either the same or different treatments; and, by virtue of comprising a disposable hydraulic circuit, requires no sterilizing equipment. In short, system 1 provides for preparing fluid as it is needed and of the required stoichiometric strength, while at the same time eliminating storage, fluid deterioration and component sterilization problems.

Finally, filter 24 provides for ensuring only perfect fluid is employed, in that any particles remaining suspended in the fluid result in clogging of filter 24 and tube 21.

Clearly, changes may be made to the embodiments of system 1 as described and illustrated herein without, however, departing from the scope of the claims of the present invention. In particular, source 19 may be replaced by a second container of given volume for compound 3, in which case, system 1 may dispense with solenoid valve 26 and weighing means 27, but will require a pump fitted along tube 18 and controlled by apparatus 7.

The above variation provides for preparing dialysis solutions involving two successive stages, the first for preparing an intermediate fluid by dissolving a first saline concentrate in water, and the second for preparing the final solution by dissolving a second concentrate in the intermediate fluid.

Heating device 29 in the Figure 1 system may be incorporated into source 19, for supplying preheated water; and the system in Figures 7 and 12 may obviously also be provided with a device 29 for heating fluid 5 and possibly also incorporated in source 19.

System 1 may also be used for preparing fluid for one or a number of treatment cycles. In particular, it may be used for preparing in one container 8 all the fluid required over a given period, e.g. one day, in which case container 8 must be so selected as to accommodate the corresponding volume of fluid when fully expanded, and the number of treatments or the maximum volume of the container must be entered as starting data on apparatus 7. Alternatively, system 1 may feature a miniaturized vibration generator for preparing fluid as it is needed.

Finally, system 1 may be designed for simultaneously preparing a given fluid in a number of containers 8 by simply using the Figure 6 embodiment of circuit 4 with a battery of bodies 73 (one for each circuit 4) all surrounded by one generator 6. Similarly, the Figure 7 system may also be used in the same way by providing a number of parallel ultrasonic generators 6'.

## Claims

**1)** A system for preparing fluid for medical treatment by dissolving a first chemical compound (2) in a second chemical compound (3); the system comprising a hydraulic circuit (4) with a container (8) to which are connected a first tube (18) by which a predetermined amount of said second compound (3) is channelled to the container (8), a second tube (21) by which, at the end of the preparation cycle, the prepared fluid is channelled from the container (8) to a user device, and a third tube (22) presenting a portion (22a) external to the container (8) and two end portions (22b, 22c) communicating with the container (8), and comprising pumping means (34) fitted to said external portion (22a), for recirculating the fluid being prepared to and from the container (8); characterized in that a predetermined amount of said first compound (2) is initially contained inside the container (8); and that a generator (6, 6') for generating vibrations of over 10 kHz frequency is fitted outside a given portion (8, 73, 81) of said circuit (4), for assisting dissolution of said first compound (2) in said second compound (3).

**2)** A system as claimed in Claim 1, characterized in that said first compound (2) may be in the form of dry or wet powder, granules or liquid; and that said second compound is liquid, and is supplied by a source (19) connected to said first tube (18); said fluid (5) also being liquid.

**3)** A system as claimed in Claim 2, characterized in that said container (8) is made of plastic material and is expansible; said container (8), in its initial configuration, presenting a portion (11) containing said first compound (2).

**4)** A system as claimed in Claim 3, characterized in that, in its initial configuration, a vacuum is formed in said container (8).

**5)** A system as claimed in Claim 3 or 4, characterized in that, during preparation of the fluid, and when fully expanded, said container (8) is box-shaped and comprises a bottom wall (9), a top wall (10), and four side walls (11), the opposite pairs of which are parallel.

**6)** A system as claimed in Claim 5, characterized in that said container (8) also presents an extension (12) formed in said top wall (10) and for connecting said third tube (22).

**7)** A system as claimed in one of the foregoing Claims, characterized in that said hydraulic circuit (4) is used for only one preparation cycle of said fluid.

**8)** A system as claimed in one of the foregoing Claims from 1 to 6, characterized in that said hydraulic circuit (4) is used for one preparation cycle, for preparing fluid for use within a given period of time.

**9)** A system as claimed in one of the foregoing Claims, characterized in that it is used for preparing fluids for infusion, haemodialysis, haemodiafiltration, haemofiltration or peritoneal dialysis.

**10)** A system as claimed in Claim 9, characterized in that, when used for preparing haemodialysis fluid, said fluid is produced by dissolving said first compound (2), defined by a given quantity of 400 to 900 grams of sodium bicarbonate, in said second compound (3) defined by 5 to 11.25 liters of water.

**11)** A system as claimed in one of the foregoing Claims, characterized in that said pumping means comprise a peristaltic pump (34) acting on said central portion (22a) of said third tube (22).

**12)** A system as claimed in one of the foregoing Claims, characterized in that said container (8) presents a first label (16) indicating in an automatically readable code the type of said compounds (2, 3) and the type of fluid to be prepared; a reading unit (17) being provided for reading said code and automatically controlling the preparation of said fluid.

**13)** A system as claimed in Claims 6 and 12, characterized in that said extension (12) comprises two side walls (13); and said container (8) presents a second label (15) indicating, in a form legible by the operator, the type of said compounds (2, 3) and the type of fluid to be prepared; said labels (15, 16) being applied to at least one of said side walls (13).

**14)** A system as claimed in Claim 12 or 13, characterized in that said reading unit (17) is connected to an electronic apparatus (7) for controlling operation of said generator (6, 6'), supply of said second compound (3) along said first tube (18), and recirculation of the fluid between said third tube (22) and said container (8).

**15)** A system as claimed in Claim 14, characterized in that said apparatus (7) comprises a central data processing unit (35) to which are connected a display (36); a data entry and control keyboard (37); a memory unit (38); a timer unit (41) for setting a minimum fluid circulating and recirculating time; a printer (42) for printing finished fluid labels; and an end-of-preparation-cycle acoustic signal (43).

**16)** A system as claimed in Claim 14 or 15, characterized in that it comprises:
a solenoid valve (26) controlled by said apparatus (7) and installed along said first tube (18), for enabling or disabling supply of said second compound (3) along said first tube (18);
means (27) for weighing said container (8) and transmitting to said apparatus (7) the weight of said container (8) together with said fluid; and
means (33, 80) for detecting characteristic parameters of the fluid, and transmitting the value of said parameters to said apparatus (7).

**17)** A system as claimed in Claims 2 and 15, characterized in that said source (19) comprises a second container containing a predetermined weight of said second compound (3) and from which said first tube (18) extends.

**18)** A system as claimed in one of the foregoing Claims, characterized in that it comprises a frame (74) supporting said container (8).

**19)** A system as claimed in one of the foregoing Claims, characterized in that it forms part of a dialysis machine; said second tube presenting a filter and being connected by a valve to the supply circuit of said machine.

**20)** A system as claimed in one of the foregoing Claims, characterized in that said generator (6) generates vibrations of 10 to 40 kHz frequency; a heating device (29) being provided for heating the fluid under the control of said apparatus (7); said heating device (29) being located adjacent to said container (8).

**21)** A system as claimed in Claim 20, characterized in that said generator (6) is located close to said container (8), for assisting dissolution inside the container (8).

**22)** A system as claimed in any one of the foregoing Claims from 1 to 20, characterized in that said circuit (4) comprises a box body (73) along said third tube (22); said generator (6) being located close to said body (73), for assisting dissolution inside the body (73).

**23)** A system as claimed in Claim 13 and one of Claims 21 and 22, characterized in that said extension (12) also comprises a top wall (14); said first tube (18) and said second tube (21) being connected to said bottom wall (9) of said container (8); and said end portions (22b, 22c) being inserted inside said container (8) through said top wall (14) of said extension (12).

**24)** A system as claimed in Claim 21, characterized in that said inlet end portion (22b) is inserted inside said container (8) to a greater length than said outlet end portion (22c).

**25)** A system as claimed in Claim 16 and one of the foregoing Claims from 20 to 24, characterized in that it comprises:
a fourth tube (28) extending from said external portion (22a);
a pump (31) fitted along said fourth tube (28) and controlled by said apparatus (7); and
a burette (32) connected to the free end of said fourth tube (28);
said detecting means (33, 80) comprising a sensor (33) for determining the conductivity and/or the pH of the fluid being prepared, and installed in said burette (32).

**26)** A system as claimed in one of the foregoing Claims from 1 to 20, characterized in that said generator is an ultrasonic generator (6') for generating vibrations of over 100 kHz frequency.

**27)** A system as claimed in Claim 26, characterized in that said ultrasonic generator (6') generates vibrations of 400 to 1000 kHz frequency.

**28)** A system as claimed in Claim 26 or 27, characterized in that said ultrasonic generator (6') comprises at least one plate (82, 83) having a number of piezoelectric ceramic disks (86) arranged in rows and columns or in offset rows.

**29)** A system as claimed in Claim 28, characterized in that said ultrasonic generator (6') contacts a flexible bag (81) located between two portions of said third tube (22).

**30)** A system as claimed in Claim 29, characterized in that said disks (86) of said ultrasonic generator (6') contact said bag (81).

**31)** A system as claimed in Claim 29, characterized in that said disks (86) of said ultrasonic generator (6') are arranged facing said bag (81); a plate (87) being arranged contacting said bag (81), for transmitting said ultrasonic vibrations from said disks (86) to said bag (81).

**32)** A system as claimed in one of the foregoing Claims from 28 to 31, characterized in that said ultrasonic generator (6') comprises two substantially parallel plates (82, 83), each presenting said number of said disks (86); said plates (82, 83) being so connected as to permit insertion between them of said bag (81); and said bag (81) being expansible so that its walls are brought into perfect contact with said ultrasonic generator (6').

**33)** A system as claimed in Claim 32, characterized in that said two plates (82, 83) are hinged to each other along a first edge (84), and connected releasably along a second edge (85) opposite said first edge (84).

**34)** A system as claimed in one of the foregoing Claims from 26 to 33, characterized in that said first tube (18) is connected to a portion (78, 79) of said third tube (22) by means of a three-way valve (77).

**35)** A system as claimed in Claims 15 and 34, characterized in that said three-way valve (77) is so controlled by said central unit (35) as first to permit water to be fed into said container (8) through said bag (81), and then to permit the fluid being prepared to be circulated between said container (8) and said bag (81).

**36)** A system as claimed in one of the foregoing Claims from 26 to 35, characterized in that said container (8) presents two chambers (8', 8'') containing two chemically incompatible compounds (2) to be dissolved in said second compound (3); said chambers (8', 8'') being connected to said end portions (22b, 22c) of said third tube (22).

**37)** A system as claimed in Claim 36, characterized in that said chambers (8', 8'') are connected to each other by a further tube (88) to which said second tube (21) is connected by means of a three-way valve (89).

**38)** A system as claimed in Claim 16 and one of the foregoing Claims from 20 to 37, characterized in that said detecting means (80) are located along said external portion (22a), and provide for detecting externally at least the colour and/or opacity of the fluid being prepared.

**39)** A process for preparing fluid for medical treatment by dissolving a first compound (2) in a second compound (3); said first compound (2) being contained inside a container (8); characterized in that it comprises, in succession, at least the following stages:
a stage wherein a predetermined amount of said second compound (3) is fed into the container (8);
a stage wherein the fluid is vibrated continually and recirculated into the container (8) along a tube (22) external to the container (8); and
a stage for checking attainment of the required characteristics of said fluid.

**40)** A process for preparing fluid for medical treatment by dissolving a first compound (2) in a second compound (3); said first compound (2) being contained inside a container (8); characterized in that it comprises, in succession, at least the following stages:
a stage wherein an electronic apparatus (7) reads a code on a label (16) applied to said container (8) and indicating said first chemical compound (2) and the fluid to be prepared;
a stage wherein, on the basis of said code reading, said apparatus (7) enables weighing means (27) and supply of said second compound (3) to said container (8);
a stage wherein, via said weighing means (27), said apparatus (7) determines whether the predetermined weight of said container (8) has been reached, for disabling supply of said second compound (3) to the container (8); and
a stage wherein said apparatus (7) enables pumping means (34) to recirculate the fluid along a tube (22) to and from said container (8); and enables a vibration generator (6, 6') generating vibrations of over 10 kHz frequency, to assist dissolution of said first compound (2) in said second compound (3).

**41)** A process as claimed in Claim 40, characterized in that it also comprises:
a stage wherein the required parameters of said fluid are entered;
a stage wherein said apparatus (7) enables detecting means (33, 80) for detecting said parameters into said fluid; and
a stage wherein said apparatus (7) compares the parameter values detected by said detecting means (33, 80) with the entered parameter values, for disabling said pumping means (34) and said generator (6, 6').

**42)** A process as claimed in Claim 40 or 41, characterized in that it also comprises:
a stage subsequent to said code reading and wherein said apparatus (7) sets a timer unit (41) to a predetermined operating time of said pumping means (34) and said generator (6, 6');
a stage wherein said apparatus (7) determines whether a predetermined time for preparing said fluid has been reached; and
a stage wherein, in the event said predetermined time has been reached, said apparatus (7) enables said detecting means (33, 80).

**43)** A process as claimed in Claim 40, wherein said electronic apparatus (7) comprises a central processing unit (35); a display (36); a keyboard (37); and a data memory (38); characterized in that it also comprises the following operations by said central processing unit (35):
an operation subsequent to said code reading and wherein said display (36) displays the reading, and requests the operator to confirm the displayed reading and continuation of the fluid preparation stage;
an operation wherein, by means of said keyboard (37), the operator either confirms continuation of the fluid preparation stage, or provides for modifying the original data shown on the display (36); and
an operation wherein said central processing unit (35) retrieves from said memory unit (38) the control data for preparing the fluid, and enables said weighing means (27) to determine the tare of said container (8).

**44)** A process as claimed in Claim 43, characterized in that it also comprises the following operations by said central processing unit (35):
an operation wherein said central processing unit (35) operates a solenoid valve (26) to enable supply of said second compound (3) to said container (8); enables said pumping means (34) to recirculate the fluid along a tube (22) external to said container (8); and enables said generator (6);
an operation wherein, via said weighing means (27), said central processing unit (35) determines whether the predetermined weight of said container (8) has been reached; and
an operation wherein said central processing unit (35) operates said solenoid valve (26) to disable supply of said second compound (3) to said container (8).

**45)** A process as claimed in Claim 44, characterized in that it also comprises the following operations:
an operation wherein said central processing unit (35) enables a timer unit (41);
an operation wherein said central processing unit (35) determines whether a predetermined time stored in said memory unit (38) has been reached;
an operation wherein, in the event said predetermined time has been reached, said central processing unit (35) enables detecting means (80) for detecting characteristic parameters of the fluid being prepared;
an operation wherein said central processing unit (35) compares the parameter values detected by said detecting means (80) with the previously adopted values of the same parameters; and
an operation wherein, in the event the parameter values detected by said detecting means (33, 80) match those adopted previously, said central processing unit (35) disables said pumping means (34) and said generator (6, 6').

**46)** A process as claimed in one of the foregoing Claims from 43 to 45, wherein said apparatus (7) also comprises a printer (42) and an acoustic signal (43); characterized in that it also comprises the following operations:
an operation wherein said central processing unit (35) enables said printer (42) to print the characteristic data of the prepared fluid on a label (68) which is then gummed to said container (8); and
an operation wherein said central processing unit (35) enables said acoustic signal (43) to inform the operator that preparation of the fluid has been completed.
